# FASCICULE DE BREVET EUROPEEN

(11) **EP 0 532 602 B1**
(45) Date de publication et mention de la délivrance du brevet: **03.08.1994**
(21) Numéro de dépôt: 91910896.9
(22) Date de dépôt: 31.05.1991
(51) Int. Cl.: C07D 277/82, C07D 417/12, A61K 31/41

(54) **DERIVES DE BENZOTHIAZOLE, LEUR PREPARATION ET LES MEDICAMENTS LES CONTENANT**
BENZOTHIAZOLINDERIVATE, VERFAHREN ZU IHRER HERSTELLUNG UND DIESE ENTHALTENDE ARZNEIMITTEL
BENZOTHIAZOLINE DERIVATIVES, PROCESS FOR THEIR PREPARATION AND DRUGS CONTAINING SAME

(30) Priorité: 07.06.1990 FR 9007068
(43) Date de publication de la demande: 24.03.1993
(73) Titulaire: RHONE-POULENC RORER S.A., 92165 Antony Cédex (FR)
(72) Inventeur: GUEREMY, Claude, F-78800 Houilles (FR); JIMONET, Patrick, F-78450 Villepreux (FR); MIGNANI, Serge, F-75014 Paris (FR)
(74) Mandataire: Savina, Jacques
(86) Numéro de dépôt international: FR9100437
(87) Numéro de publication internationale: WO9118892

(56) Documents cités:
- EP-A- 0 050 551
- EP-A- 0 356 234
- EP-A- 0 374 040

## Description

EP-A-50551 décrit l'amino-2 trifluorométhoxy-6 benzothiazole comme base de médicaments anticonvul- sivants.

La présente invention concerne des composés de formule : 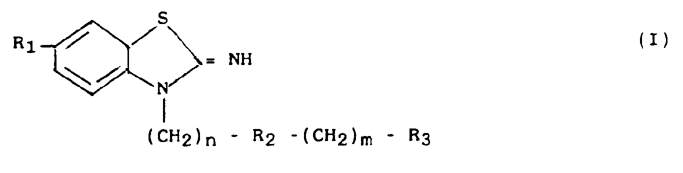 leurs sels, leurs procédés de préparation et les médicaments les contenant.

Dans la formule (I),
- R₁ représente un radical polyfluoroalcoxy,
- R₂ représente un atome de soufre ou d'azote substitué par un radical alkyle ou un radical sulfonyle, ou sulfinyle,
- R₃ représente un radical phényle, benzoyle, -NR₄R₅ ou pipéridinyl-4 substitué en position-1 par un radical phénylalkyle,
- R₄ représente un radical alkyle,
- R₅ représente un radical phénylalkyle,
- n est égal a 1, 2 ou 3,
- m est égal à 0, 1, 2 ou 3.

Dans les définitions qui précèdent et celles qui seront citées ci-après, les radicaux alkyle et alcoxy et les portions alkyle et alcoxy contiennent 1 à 4 atomes de carbone en chaîne droite ou ramifiée.

Les radicaux polyfluoroalcoxy sont de préférence les radicaux trifluorométhoxy, pentafluoroéthoxy, tri- fluoro -2,2,2 éthoxy ou tétrafluoro -1,1,2,2 éthoxy.

L'invention concerne également les sels d'addition des composés de formule (I) avec les acides minéraux ou organiques.

Les composés de formule (I) pour lesquels R₂ représente un atome de soufre peuvent être préparés par hydrolyse d'un dérivé de formule : 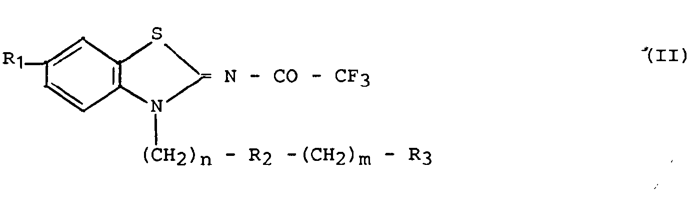 dans laquelle R_{1'} R₃, n et m ont les mêmes significations que dans la formule (I) et R₂ représente un atome de soufre.

Cette hydrolyse s'effectue généralement au moyen d'une base telle qu'un carbonate de métal alcalin (sodium, potassium de préférence) ou l'ammoniaque concentrée, au sein d'un mélange eau-alcool, à une température comprise entre 20°C et la température d'ébullition du solvant.

Les dérivé de formule (II) peuvent être obtenus par action d'un sulfure de formule : 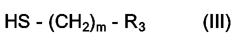 dans laquelle m et R₃ ont les mêmes significations que dans la formule (I) sur un dérivé de formule : 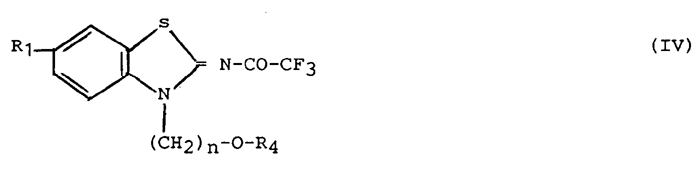 dans laquelle R₁ et n ont les mêmes significations que dans la formule (I) et R₄ représente un groupe réactif tel qu'un radical méthanesulfonyle ou p-toluènesulfonyle.

Cette réaction s'effectue au moyen d'une base telle qu'un hydrure de métal alcalin (sodium de préférence), au sein d'un solvant inerte tel que le diméthylformamide à une température voisine de 25°C.

Les dérivés de formule (III) pour lesquels R₃ représente un radical pipéridinyl-4 substitué en position-1 par un radical phénylalkyle peuvent être préparés par application ou adaptation de la méthode décrite dans les exemples.

Les dérivés de formule (IV) peuvent être préparés par action d'un dérivé de formule : 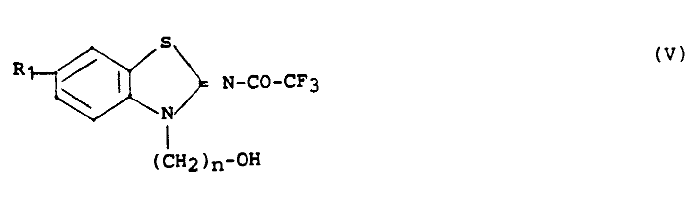 dans laquelle R₁ et n ont les mêmes significations que dans la formule (I), sur le chlorure d'acide méthane- sulfonique ou p-toluènesulfonique, soit au sein d'un solvant inerte tel qu'un solvant aromatique (benzène, toluène, xylène par exemple) ou un solvant chloré (chloroforme, chlorure de méthylène par exemple), en présence d'une amine tertiaire telle que la triéthylamine, à une température voisine de 20°C, soit au sein de la pyridine, à une température voisine de 0°C.

Les dérivés de formule (V) peuvent être obtenus par action de trifluoroacétate d'éthyle sur un dérivé de formule : 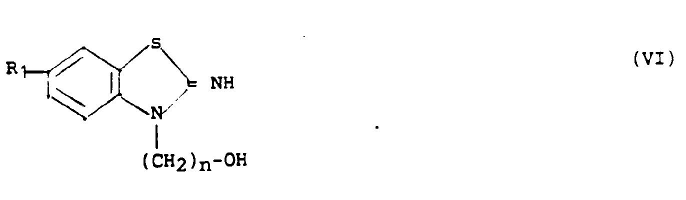 dans laquelle R₁ et n ont les mêmes significations que dans la formule (I).

Cette réaction s'effectue généralement au sein d'un alcool (méthanol, éthanol par exemple), en présence d'une amine tertiaire telle que la triéthylamine, à une température voisine de 20°C.

Les dérivés de formule (VI) peuvent être préparés par action d'un dérivé halogéné de formule : 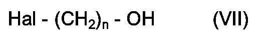 dans laquelle n a les mêmes significations que dans la formule (I) et Hal représente un atome d'halogène (brome ou chlore de préférence) sur un amino-2 polyfluoroalcoxy-6 benzothiazole.

Cette réaction s'effectue au sein d'un alcool (éthanol, méthanol de préférence) à la température d'ébullition du solvant.

Les amino-2 polyfluoroalcoxy-6 benzothiazoles peuvent être préparés par application ou adaptation de la méthode décrite par L.M. YAGUPOL'SKII et coll. , Zh. Obshch. Khim., 33(7), 2301 (1963).

Les composés de formule (I) pour lesquels R₂ représente un atome de soufre peuvent également être obtenus par action d'un amino-2 polyfluoroalcoxy-6 benzothiazole sur un dérivé de formule : 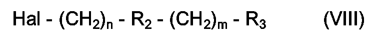 dans laquelle n, m et R₃ ont les mêmes significations que dans la formule (I), R₂ représente un atome de soufre et Hal représente un atome d'halogène (chlore et brome de préférence).

Cette réaction s'effectue généralement au sein d'un solvant inerte tel qu'un alcool(méthanol, éthanol par exemple) ou une cétone (méthyléthylcétone par exemple), à une température comprise entre 20°C et la température d'ébullition du solvant.

Les dérivés de formule (VIII) peuvent être obtenus par application ou adaptation de la méthode décrite par KULKA et Coll.,Can. J. Chem., 35, 519 (1957), ou par halogénation d'un dérivé de formule : 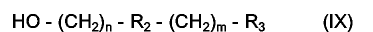 dans laquelle n, m et R₃ ont les mêmes significations que dans la formule (I) et R₂ représente un atome de soufre.

Cette réaction s'effectue au moyen d'un agent halogénant (chlorure de thionyle, bromure de thionyle par exemple), au sein d'un éther (éther diéthylique par exemple), à la température d'ébullition du solvant.

Les dérivés de formule (IX) peuvent être obtenus par action d'un dérivé de formule : 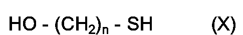 dans laquelle n a les mêmes significations que dans la formule (I), sur un dérivé de formule : 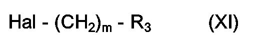 dans laquelle m et R₃ ont les mêmes significations que dans la formule (I) et Hal représente un atome d'halogène (chlore ou brome de préférence).

Cette réaction s'effectue au moyen d'une base telle qu'un alcoolate de métal alcalin (éthylate de sodium ou de potassium, méthylate de sodium ou de potassium par exemple), au sein d'un solvant inerte tel qu'un alcool, à la température d'ébullition du solvant.

Les composés de formule (I) pour lesquels R₂ représente un radical sulfonyle ou sulfinyle peuvent être obtenus par oxydation des dérivés de formule (I) correspondants pour lesquels R₂ représente un atome de soufre.

Cette oxydation s'effectue généralement au moyen d'acide m-chloroperbenzoïque au sein d'un solvant inerte tel qu'un alcool (méthanol ou éthanol par exemple), un solvant chloré (chlorure de méthylène ou chloroforme par exemple), à une température comprise entre -20°C et 30°C.

Les composés de formule (I) pour lesquels R₂ représente un atome d'azote substitué par un radical alkyle peuvent être préparés par action de brome et d'un thiocyanate de métal alcalin sur un dérivé de formule : 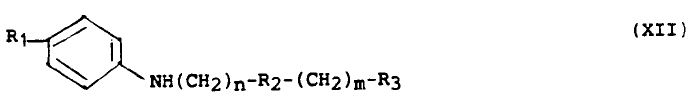 dans laquelle R_{1'} R₃, n et m ont les mêmes significations que dans la formule (I) et R₂ représente un atome d'azote substitué par un radical alkyle.

Cette réaction s'effectue de préférence au sein de l'acide acétique, à une température voisine de 20°C.

Comme thiocyanate de métal alcalin, on utilise de préférence le thiocyanate de potassium.

Les dérivés de formule (XII) peuvent être obtenus par action d'une amine de formule : 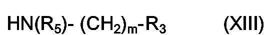 dans laquelle R₅ représente un radical alkyle et R₃ et m ont les mêmes significations que dans la formule (I) sur un dérivé de formule : 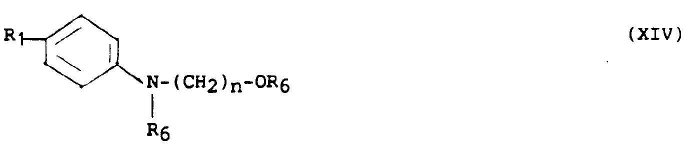 dans laquelle R₁ et n ont les mêmes significations que dans la formule (I) et R₆ représente un radical p-toluènesulfonyle.

Cette réaction s'effectue généralement en présence d'hydrogénocarbonate de sodium, au sein d'un solvant inerte tel que le diméthylformamide, à une température comprise entre 50°C et 100°C.

Les dérivés de formule (XIV) peuvent être obtenus par action de chlorure de p-toluènesulfonyle sur un dérivé de formule : 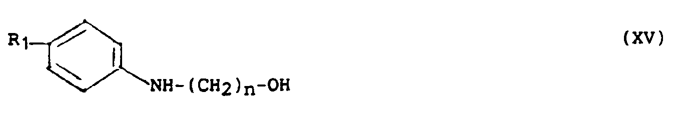 dans laquelle R₁ et n ont les mêmes significations que dans la formule (I).

Cette réaction s'effectue généralement au sein d'un solvant inerte tel qu'un solvant chloré (chloroforme, chlorure de méthylène par exemple) en présence d'une amine tertiaire telle que la triéthylamine, à une température comprise entre 0°C et 30°C.

Les dérivés de formule (XV) peuvent être obtenus par action d'une polyfluoroalcoxy-4 aniline sur un dérivé de formule (VII).

Cette réaction s'effectue généralement à une température comprise entre 100°C et 170°C.

Les mélanges réactionnels obtenus par les divers procédés décrits précédemment sont traités suivant des méthodes classiques physiques (évaporation, extraction, distillation, cristallisation, chromatographie...) ou chimiques (formation de sels...).

Les composés de formule (I), sous forme de base libre, peuvent être éventuellement transformés en sels d'addition avec un acide minéral ou organique par action d'un tel acide au sein d'un solvant organique tel qu'un alcool, une cétone, un éther ou un solvant chloré.

Les composés de formule (I) et leurs sels présentent des propriétés pharmacologiques intéressantes. Ces composés sont actifs vis-à-vis des convulsions induites par le glutamate et sont donc utiles dans le traitement et la prévention des phénomènes convulsifs, des troubles schizophréniques et notamment des formes déficitaires de la schizophrénie, des troubles du sommeil, des phénomènes liés à l'ischémie cérébrale ainsi que les affections neurologiques où le glutamate peut être impliqué telles que la maladie d'Alzheimer, la maladie d'Huntington, la sclérose amyotrophique latérale et l'atrophie olivopontocérébelleuse.

L'activité des composés de formule (I) vis-à-vis des convulsions induites par le glutamate a été déterminée selon une technique inspirée de celle de I.P. LAPIN, J. Neural. Transmission, vol. 54, 229-238 (1982) ; l'injection du glutamate par voie intracérébroventriculaire étant effectuée selon une technique inspirée de celle de R. CHERMAT et P. SIMON, J. Pharmacol. (Paris), vol. 6, 489-492 (1975). Leur DE₅₀ est inférieure ou égale à 10mg/kg.

Les composés de formule (I) présentent une toxicité faible. Leur DL₅₀ est supérieure à 15 mg/kg par voie l.P chez la souris.

Pour l'emploi médicinal, il peut être fait usage des composés de formule (I) tels quels ou à l'état de sels pharmaceutiquement acceptables, c'est-à-dire non toxiques aux doses d'utilisation.

Comme exemples de sels pharmaceutiquement acceptables, peuvent être cités les sels d'addition avec les acides minéraux ou organiques tels que acétate, propionate, succinate, benzoate, fumarate, maléate, oxalate, méthanesulfonate, iséthionate, théophyllineacétate, salicylate, phénolphtalinate, méthylène-bis-B-oxy- naphtoate, chlorhydrate, sulfate, nitrate et phosphate.

Les exemples suivants montrent comment l'invention peut être mise en pratique.

### EXEMPLE 1

A une suspension de 0,75 g d'hydrure de sodium à 50 % en dispersion dans l'huile de vaseline, on ajoute une solution de 2,7 g de (N-benzyl N-méthylamino)-2 éthanethiol dans 15 cm³ de diméthylformamide. Le milieu réactionnel est agité 1 heure à 25°C. On y additionne par petites fractions 8 g de p-toluènesulfonate de (trifluoroacétylimino-2 trifluorométhoxy-6 benzothiazolinyl-3)-2 éthyle. On agite 12 heures à 25°C. A la solution de trifluoroacétylimino-2 {[(N-méthylbenzylamino)-2 éthylthio]-2 éthyl}-3 trifluorométhoxy-6 benzothiazoline ainsi obtenue, on ajoute 40 cm³ d'éthanol, 8 cm³ d'eau et 15 cm³ d'ammoniaque concentrée (10N) et porte au reflux pendant 1 heure. Après refroidissement à 25°C, on extrait par 2 fois 100 cm³ d'éther diéthylique. Les phases organiques réunies sont séchées sur sulfate de magnésium anhydre et concentrées à 40°C sous pression réduite (20 mm de mercure 2,7 kPa). L'huile obtenue (8 g) est purifiée par flash-chromatographie sur colonne de silice, sous courant d'azote à moyenne pression (0,5-1,5 bar) avec un mélange acétate d'éthyle-cyclohexane (90-10 en volumes) comme éluant. On isole 2,7 g d'une huile qui par action d'acide oxalique dans l'acétone conduit à 3,5 g de dioxalate d'imino-2 {[(N-méthylbenzylamino)-2 éthylthio]-2 éthyl}-3 trifluorométhoxy-6 benzothiazoline fondant à 205°C.

Le p-toluènesulfonate de (trifluoroacétylimino-2 trifluorométhoxy-6 benzothiazolinyl-3)-2 éthyle peut être préparé selon le procédé suivant : 19,3 g de (trifluoroacétylimino-2 trifluorométhoxy-6 benzothiazolinyl-3)-2 éthanol sont ajoutés progressivement à 19,7 g de chlorure de p-toluènesulfonyle en solution dans 120 cm³ de pyridine refroidie à 0°C. La réaction est poursuivie 1 heure à 10-15°C. Le milieu réactionnel est ajouté à 500 cm³ d'eau distillée et la phase organique extraite par 3 fois 100 cm³ de dichloro- méthane. Après lavage par 2 fois 50 cm³ d'acide chlorhydrique 1 N, puis 2 fois 50 cm³ d'eau distillée, séchage sur sulfate de magnésium et concentration à sec sous pression réduite (20 mm de mercure ; 2,7 kPa), on obtient 14,1 g de p-toluènesulfonate de (trifluoroacétylimino-2 trifluorométhoxy-6 benzothiazolinyl-3)-2 éthanol fondant à 143°C.

Le (trifluoroacétylimino-2 trifluorométhoxy-6 benzothiazolinyl-3)-2 éthanol peut être préparé de la manière suivante : 20,7 g de bromhydrate d'(imino-2 trifluorométhoxy-6 benzothiazolinyl-3)-2 éthanol, 9,8 g de trifluoroacétate d'éthyle et 16,1 cm³ de triéthylamine sont agités dans 100 cm³ d'éthanol pendant 22 heures à une température voisine de 20°C. Après concentration à sec sous pression réduite, le résidu obtenu est purifié par chromatographie sur colonne de silice avec de l'acétate d'éthyle comme éluant. On obtient 19,2 g de (trifluoroacétylimino-2 trifluorométhoxy-6 benzothiazolinyl-3)-2 éthanol fondant à 144°C.

L'(imino-2 trifluorométhoxy-6 benzothiazolinyl-3)-2 éthanol peut être préparé selon le procédé suivant : 9,4 g d'amino-2 trifluorométhoxy-6 benzothiazole et 10 g de bromo-2 éthanol dans 30 cm³ d'éthanol absolu sont chauffés pendant 95 heures à ébullition. Le mélange est ensuite refroidi à une température voisine de 20°C. Le précipité formé est filtré et lavé avec 100 cm³ d'éther éthylique. On obtient 6,4 g de bromhydrate d'(imino-2 trifluorométhoxy-6 benzothiazolinyl-3)-2 éthanol fondant à 219°C.

L'amino-2 trifluorométhoxy-6 benzothiazole peut être préparé selon la méthode décrite par L.M. YAGU-POL'SKII et Coll.; Zh. Obshch. Khim, 33(7), 2301 (1963).

### EXEMPLE 2

On opère comme dans l'exemple 1, à partir de 1,4 g d'hydrure de sodium à 50 % en dispersion dans l'huile de vaseline dans 30 cm³ de diméthylformamide et de 5,4 g de benzyl-1 mercapto-4 pipéridine dans 26 cm3 de diméthylformamide.On laisse le tout 1 heure à une température voisine de 20°C. A cette suspension on ajoute, pendant une demi-heure, 13,7 g de para-toluènesulfonate de (trifluoroacétylimino-2 trifluorométhoxy-6 benzothiazolinyl-3)-2 éthyle et on laisse le tout une nuit à une température voisine de 20°C sous agitation. On ajoute ensuite 500 cm³ d'eau, extrait la solution aqueuse par deux fois 70 cm³ d'acétate d'éthyle, sèche sur sulfate de magnésium anhydre, filtre et concentre à sec à 40°C sous pression réduite (20 mm de mercure ; 2,7 kPa). L'huile ainsi obtenue est purifiée parflash-chromatographie surcolonne de silice, sous courantd'azote à moyenne pression (0,5-1,5 bar) avec de l'acétate d'éthyle comme éluant. On isole ainsi 4,3 g d'un solide beige qui est introduit dans une solution de 172 cm³ de méthanol et 51,7 cm³ d'une solution aqueuse de carbonate de potassium à 7 %. On laisse 12 heures à 25°C et concentre à sec à 70°C sous pression réduite (20 mm de mercure ; 2,7 kPa). On ajoute 220 cm³ d'eau et 110 cm³ d'acétate d'éthyle. La phase organique est séparée, séchée sur sulfate de magnésium anhydre et concentrée à sec à 40°C sous pression réduite (20 mm de mercure ; 2,7 kPa). On isole ainsi 3,3 g d'une huile jaune qui est directement reprise par 3 cm³ d'éther chlorhydrique 4N et 100 cm³ d'éther diéthylique. On isole ainsi 2,4 g d' i mino-2 {[(benzyt-1 pipéridinyl-4)thio]-2 éthyl}-3 trifluorométhoxy-6 benzothiazoline sous forme de dichlorhydrate fondant à 200°C.

La benzyl-1 mercapto-4 pipéridine peut être préparée de la façon suivante : 8 g de benzyl-1 acétylthio-4 pipéridine dissous dans 85 cm³ d'une solution aqueuse de soude à 6 % sont agités, sous courant d'azote, pendant 16 heures. A cette solution on ajoute 10 cm³ d'acide acétique et 57,5 g de sulfate d'ammonium. On extrait ensuite par deux fois 100 cm³ d'éther diéthylique, sèche la phase organique sur du sulfate de magnésium anhydre et concentre à sec à 40°C sous pression réduite (20 mm de mercure ; 2,7 kPa). On isole ainsi directement 5,5 g de benzyl-1 mercapto-4 pipéridine sous forme d'une huile utilisée telle quelle dans les synthèses ultérieures.

La benzyl-1 acétylthio-4 pipéridine peut être préparée de la façon suivante : à 20,8 g de triphénylphosphine dissous dans un mélange de 122 cm³ de tétrahydrofuranne et de 0,12 cm³ de diméthylformamide on ajoute sous forte agitation, à -5°C et sous courant d'azote, 16,1 g d'azido carboxylate de diisopropyle pendant 10 minutes. Puis à 5°C on additionne 6,1 g de benzyl-1 hydroxy-4 pipéridine.

On porte le tout à 50°C pendant 2 heures. On filtre ensuite l'oxyde de triphé- nylphosphine ainsi formé et on lave la solution organique par 2 fois 40 cm³ d'eau. La phase organique est concentrée à sec sous pression réduite (20 mm de mercure ; 2,7 kPa). On isole ainsi 12,35 g d'une huile qui est purifiée parflash chromatographie sur colonne de silice, sous courant d'azote, à moyenne pression (0,5-1,5 bar) avec un mélange cyclohexane-acétate d'éthyle (70-30 en volumes) comme éluant. On obtient ainsi 8 g de benzyl-1 acétylthio-4 pipéridine sous forme d'huile utilisée telle quelle dans les synthèses ultérieures.

### EXEMPLE 3

A une solution de 1,9 g d'imino-2 {[(benzyl-1 pipéridinyl-4) thio]-2 éthyl}-3 trifluorométhoxy-6 benzothiazoline dichlorhydrate dissous dans 50 cm3 d'un mélange de dioxane et d'eau (50-50 en volumes) à température voisine de 20°C, on ajoute en 10 minutes, 0,86 g d'acide méta-chloroperbenzoïque à 75 %. On agite à 25°C pendant 12 heures puis ajoute 100 cm³ d'eau et 10 cm³ d'une solution aqueuse de soude 1 N. On extrait par deux fois 100 cm³ d'acétate d'éthyle puis sèche la phase organique sur sulfate de magnésium anhydre et la concentre à sec à 40°C sous pression réduite (20 mm de mercure ; 2,7 kPa). On isole 1,5 g d'une huile incolore qui est cristallisée dans 20 cm³ d'éther diéthylique. On obtient ainsi 0,7 g d'imino-2 {[(benzyl-1 pipéridinyl-4) sulfinyl]-2 éthyl}-3 trifluorométhoxy-6 benzothiazoline fondant à 97°C.

### EXEMPLE 4

A 1,42 g d'[(imino-2 trifluorométhoxy-6 benzothiazolinyl-3) -2 éthylthio]-4 butyrophénone en solution dans 20 cm³ d'éthanol absolu refroidi à -35°C, on ajoute, en environ 15 minutes, 0,74 g d'acide m-chloroperbenzoïque à 75 %. La réaction est poursuivie 1 heure à cette température. Le milieu réactionnel est ajouté à 50 cm³ d'éther éthylique et le chlorhydrate formé par addition de 0,76 cm³ d'éther chlorhydrique 4,2N. Le précipité est filtré, puis recristallisé dans le méthanol. On obtient 0,7 g de chlorhydrate d'[(imino-2 trifluorométhoxy-6 benzothiazolinyl-3)-2 éthylsulfinyl]-4 butyrophénone-(RS) sublimant à 170°C.

L'[(imino-2 trifluorométhoxy-6 benzothiazolinyl-3)-2 éthylthio]-4 butyrophénone peut être préparé de la façon suivante : 9,36 g d'amino-2 trifluorométhoxy-6 benzothiazole et 10,68 g de (chloro-2 éthylthio)-4 butyrophénone dans 40 cm³ de méthyléthylcétone sont chauffés à ébullition pendant 72 heures. Après refroidissement à une température voisine de 20°C, le milieu réactionnel est concentré à sec sous pression réduite. Le résidu est repris dans l'eau distillée et la phase organique extraite par du dichlorométhane après neutralisation par de la soude 1 N. Le produit brut obtenu après traitement habituel est purifié par chromatographie sur colonne de silice avec un mélange acétate d'éthyle-cyclohexane (30-70 en volumes) comme éluant. On obtient, après recristallisation dans le cyclohexane, 3,67 g d'[(imino-2 trifluorométhoxy-6 benzothiazolinyl-3)-2 éthyl- thio]-4 butyrophénone fondant à 72°C.

La (chloro-2 éthylthio)-4 butyrophénone peut être préparée selon le procédé suivant : à 29,46 g d'(hydroxy-2 éthylthio)-4 butyrophénone dans 80 cm³ d'éther éthylique, on ajoute goutte à goutte 11,78 cm³ de chlorure de thionyle à 0°C. Le milieu réactionnel est porté à ébullition pendant 1 heure. Après refroidissement à une température voisine de 20°C et concentration à sec sous pression réduite, le produit brut est repris 2 fois dans 20 cm³ d'éthanol et concentré à sec pour être utilisé brut dans la réaction suivante.

L'(hydroxy-2 éthylthio)-4 butyrophénone peut être préparée selon le procédé suivant : à 6,06 g de sodium dans 170 cm³ d'éthanol absolu sous azote à une température voisine de 20°C, on ajoute après solubilisation totale, 18,56 cm³ de mercapto-2 éthanol en environ 30 minutes, puis 42,25 cm³ de chloro-4 butyrophénone en environ 30 minutes. Le milieu réactionnel est porté à ébullition pendant 2 heures. Après refroidissement à une température voisine de 20°C, le précipité formé est filtré et le filtrat concentré à sec sous pression réduite. Le produit brut est purifié par chromatographie sur colonne de silice avec un mélange d'acétate d'éthyle et de cyclohexane (50-50 en volumes) comme éluant. On obtient 29,46 g d'(hydroxy-2 éthylthio)-4 butyrophénone sous forme d'une huile jaune utilisée telle quelle dans les synthèses ultérieures.

L'amino-2 trifluorométhoxy-6 benzothiazole peut être obtenu selon le procédé décrit par L.M. YAGU-POL'SXII et Coll., Zh. Obshch. Khim., 33(7), 2301 (1963).

### EXEMPLE 5

On opère comme dans l'exemple 4 à partir de 2,3 g de chlorhydrate d'imino-2 [(phényl-3 propylthio)-2 éthyl]-3 trifluorométhoxy-6 benzothiazoline et de 1,18 g d'acide m-chloroperbenzoïque à 75 % dans 30 cm³ d'éthanol absolu. Après neutralisation par de la soude 1N, le produit brut est purifié par chromatographie sur colonne de silice avec un mélange acétate d'éthyle-méthanol (90-10 en volumes) comme éluant. Après transformation en chlorhydrate par addition d'éther chlorhydrique 4,2 N dans l'acétate d'éthyle et recristallisation dans un mélange d'acétone et d'eau (95-5 en volumes), on obtient 0,65 g de chlorhydrate d'imino-2 [(phényl-3 propylsulfinyl)-2 éthyl]-3 trifluorométhoxy-6 benzothiazoline-(RS) fondant à 165°C.

Le chlorhydrate d'imino-2 [(phényl-3 propylthio)-2 éthyl]-3 trifluorométhoxy-6 benzothiazoline peut être préparé selon le procédé suivant : on opère comme dans l'exemple 4, mais à partir de 9,5 g d'amino-2 trifluorométhoxy-6 benzothiazole et de 9,5 g de (chloro-2 éthylthio)-1 phényl-3 propane dans 40 cm³ de méthyléthylcétone. Après recristallisation dans l'acétonitrile, on obtient4,7 g de chlorhydrate d'imino-2 [(phényl-3 pro- pylthio)-2 éthyl]-3 trifluorométhoxy-6 benzothiazoline fondant à 146°C.

Le (chloro-2 éthylthio)-1 phényl-3 propane peut être préparé selon la méthode décrite par M. KULKA et F.G. VAN STRYK, Can.J.Chem., 35, 519 (1957).

### EXEMPLE 6

3,0 g de N-méthyl N-[(trifluorométhoxy-4 anilino)-2 éthyl]-benzylamine et 3,6 g de thiocyanate de potassium dans 30 cm³ d'acide acétique sont traités goutte à goutte par 1,47 g de brome en solution dans 17 cm³ d'acide acétique à une température voisine de 20°C. La réaction est poursuivie 18 heures à cette température. Après addition de 250 cm³ d'eau distillée, le milieu réactionnel est neutralisé par de la soude à 30 % et la phase organique extraite par 2 fois 150 cm³ d'acétate d'éthyle, séchée sur sulfate de magnésium et concentrée à sec sous pression réduite (20 mm de mercure ; 2,7 kPa). Le résidu obtenu est purifié par chromatographie sur colonne de silice avec de l'acétate d'éthyle comme éluant. Après formation du chlorhydrate par addition de 0,9 cm³ d'éther chlorhydrique 4,2N dans 20 cm³ d'acétate d'éthyle, on obtient 0,96 g de chlorhydrate de (N-benzyl N-méthyl-amino-2 éthyl)-3 imino-2 trifluorométhoxy-6 benzothiazoline fondant à 180°C.

La N-méthyl N-[(ttifiuorométhoxy-4aniiino)-2éthyi]-benzyiamine peut être préparée de la façon suivante : un mélange de 6,0 g de p-toluènesulfonate de N-p-toluènesulfonyl (trifluorométhoxy-4 anilino)-2 éthyle, 1,51 g de N-méthylbenzylamine et 1,05 g d'hydrogénocarbonate de sodium dans 100 cm³ de diméthylformamide est chauffé 19 heures à 80°C. Après refroidissement à une température voisine de 20°C, le milieu réactionnel est concentré à sec sous pression réduite (7 mm de mercure : 0,95 kPa). Le résidu est lavé 2 fois par 100 cm³ d'eau distillée puis repris par 40 cm3 d'éthanol absolu et concentré à sec sous pression réduite. Le produit brut est traité par 26 cm³ d'acide chlorhydrique à 37 % dans un mélange d'acide acétique (26 cm³) et d'eau distillée (17 cm³). Le mélange est chauffé 19 heures à ébullition. Après refroidissement à une température voisine de 20°C et addition à 300 cm³ d'eau distillée, la solution aqueuse est neutralisée par de la soude à 30 % et la phase organique extraite par de l'acétate d'éthyle. On obtient 3,34 g de N-méthyl N-[(trifluorométhoxy-4 anilino)-2 éthyl]-benzylamine sous forme d'une huile utilisée à l'état brut dans la réaction suivante.

Le p-toluènesulfonate de N-p-toluènesulfonyl (trifluorométhoxy-4 anilino)-2 éthyle peut être préparé selon le procédé suivant : à 5,0 g de (trifluorométhoxy-4 anilino)-2 éthanol et 6,35 cm³ de triéthylamine dans 50 cm³ de dichlorométhane à 0°C, on ajoute progressivement 8,6 g de chlorure de p-toluènesulfonyle. La réaction est poursuivie 2 heures à une température voisine de 20°C, puis le milieu réactionnel lavé 3 fois par 50 cm³ d'eau distillée.

La phase organique est séchée sur sulfate de magnésium et concentrée à sec sous pression réduite (20 mm de mercure 2,7 kPa). Après addition de 50 cm³ d'éthanol absolu, le précipité formé est filtré. On obtient 7,3 g de p-toluènesulfonate de N-p-toluènesulfonyl (trifluorométhoxy-4 anilino)-2 éthyle fondant à 88°C.

Le (trifluorométhoxy-4 anilino)-2 éthanol peut être préparé de la manière suivante : 88,5 g de trifluorométhoxy-4 aniline et 31,2 g de bromo-2 éthanol sont chauffés à 160°C pendant 1,5 heures. Après refroidissement à une température voisine de 20°C, le milieu réactionnel est repris dans 200 cm³ de dichlorométhane, l'insoluble filtré et le filtrat concentré à sec sous pression réduite. Après purification par chromatographie sur colonne de silice avec un mélange acétate d'éthyle-cyclohexane (40-60 en volumes) comme éluant, on obtient 26,8 g de (trifluorométhoxy-4 anilino)-2 éthanol sous forme d'une huile orangée.

### EXEMPLE 7

En opérant comme à l'exemple 6 mais à partir de 3,92 g de N-(phényl-2 éthylamine) N-[(trifluorométhoxy-4 anilino)-2 éthyl] méthylamine, 4,39 g de thiocyanate de potassium et 1,65 g de brome dans 60 cm³ d'acide acétique, on obtient 0,36 g de dichlorhydrate d'imino-2 [N-méthyl phényl-2 éthylamino)-2 éthyl]-3 trifluorométhoxy-6 benzothiazoline fondant à 216°C.

La N-(phényl-2 éthylamine) N-[(trifluorométhoxy-4 ani lino)-2 éthyl]méthylamine peut être préparée de la façon suivante : on opère comme à l'exemple 6 mais à partir de 6 g de p-toluènesulfonate de N-p-toluènesulfonyl (trifluorométhoxy-4 anilino)-2 éthyle, 1,68 g de N-méthyl phényl-2 éthylamine et de 1,05 g d'hydrogéno- carbonate de sodium dans 100 cm³ de diméthylformamide. Après traitement par 26 cm³ d'acide-chlorhydrique à 37 %, on obtient 3,92 g de N-(phényl-2 éthylamine) N-[(trifluorométhoxy-4 anilino)-2 éthyl]-méthylamine sous forme d'une huile utilisée brute dans la réaction suivante.

La présente invention concerne également les médicaments constitués par un composé de formule (I) ou un sel d'un tel composé à l'état pur ou sous forme d'une composition dans laquelle il est associé à tout autre produit pharmaceutiquement compatible, pouvant être inerte ou physiologiquement actif. Les médicaments selon l'invention peuvent être employés par voie orale, parentérale, rectale ou topique.

Comme compositions solides pour administration orale, peuvent être utilisés des comprimés, pilules, poudres (capsules de gélatine, cachets) ou granulés. Dans ces compositions, le principe actif selon l'invention est mélangé à un ou plusieurs diluants inertes, tels que amidon, cellulose, saccharose, lactose ou silice.

Ces compositions peuvent également comprendre des substances autres que les diluants, par exemple un ou plusieurs lubrifiants tels que le stéarate de magnésium ou le talc, un colorant, un enrobage (dragées) ou un vernis.

Comme compositions liquides pour administration orale, on peut utiliser des solutions, des suspensions, des émulsions, des sirops et des élixirs pharmaceutiquement acceptables contenant des diluants inertes tels que l'eau, l'éthanol, le glycérol, les huiles végétales ou l'huile de paraffine. Ces compositions peuvent comprendre des substances autres que les diluants, par exemple des produits mouillants, édulcorants, épaississants, aromatisants ou stabilisants.

Les compositions stériles pour administration parentérale, peuvent être de préférence des solutions aqueuses ou non aqueuses, des suspensions ou des émulsions. Comme solvant ou véhicule, on peut employer l'eau, le propylèneglycol, un polyéthylèneglycol, des huiles végétales, en particulier l'huile d'olive, des esters organiques injectables, par exemple l'oléate d'éthyle ou autres solvants organiques convenables. Ces compositions peuvent également contenir des adjuvants, en particulier des agents mouillants, isotonisants, émulsifiants, dispersants et stabilisants. La stérilisation peut se faire de plusieurs façons, par exemple parfiltration aseptisante, en incorporant à la composition des agents stérilisants, par irradiation ou par chauffage. Elles peuvent également être préparées sous forme de compositions solides stériles qui peuvent être dissoutes au moment de l'emploi dans de l'eau stérile ou tout autre milieu stérile injectable.

Les compositions pour administration rectale sont les suppositoires ou les capsules rectales qui contiennent, outre le produit actif, des excipients tels que le beurre de cacao, des glycérides semi-synthétiques ou des polyéthylèneglycols.

Les compositions pour administration topique peuvent être par exemple des crèmes, pommades, lotions, collyres, collutoires, gouttes nasales ou aérosols.

En thérapeutique humaine, les composés selon l'invention sont particulièrement utiles pour le traitement et la prévention des phénomènes convulsifs, des troubles schizophréniques et notamment des formes déficitaires de la schizophrénie, des troubles du sommeil, des phénomènes liés à l'ischémie cérébrale et des affections neurologiques où le glutamate peut être impliqué telles que la maladie d'Alzheimer, la maladie d'Hun- tington, la sclérose amyotrophique latérale et l'atrophie olivopontocérébelleuse.

Les doses dépendent de l'effet recherché, de la durée du traitement et de la voie d'administration utilisée ; elles sont généralement comprises entre 30 et 300 mg par jour par voie orale pour un adulte avec des doses unitaires allant de 10 à 100 mg de substance active.

D'une façon générale, le médecin déterminera la posologie appropriée en fonction de l'âge, du poids et de tous les autres facteurs propres au sujet à traiter.

Les exemples suivants illustrent des compositions selon l'invention :

### EXEMPLE A

On prépare, selon la technique habituelle, des gélules dosées à 50 mg de produit actif ayant la composition suivante :
- imino-2 {[(N-méthylbenzylamino)-2 éthylthio]-2 éthyl}-3 trifluorométhoxy-6 benzothiazoline 50 mg
- cellulose 18 mg
- lactose 55 mg
- silice colloïdale 1 mg
- carboxyméthylamidon sodique 10 mg
- talc 10 mg
- stéarate de magnésium 1 mg

### EXEMPLE B

On prépare, selon la technique habituelle, des oomprimés dosés à 50 mg de produit actif ayant la oom- position habituelle :
- imino-2 {[(benzyl-1 pipéridinyl-4) sulfinyl]-2 éthyl}-3 trifluorométhoxy-6 benzothiazoline 50 mg
- lactose 104 mg
- cellulose 40 mg
- polyvidone 10 mg
- carboxyméthylamidon sodique 22 mg
- talc 10 mg
- stéarate de magnésium 2 mg
- silice colloïdale 2 mg
- mélange d'hydroxyméthylcellulose, glycérine, oxyde de titane (71-3,5-24,5) q.s.p. 1 comprimé pelliculé terminé à 245 mg

### EXEMPLE C

On prépare une solution injectable contenant 10 mg de produit actif ayant la composition suivante :
- Immino-2{«N-méthylbenzylamino)-2 éthylthio]-2 ethyl}-3 trifluorométhoxy-6 benzothiazoline 10 mg
- acide benzoïque 80 mg
- alcool benzylique 0,06 cm³
- benzoate de sodium 80 mg
- éthanol à 95 % 0,4 cm³
- hydroxyde de sodium 24 mg
- propylène glycol 1,6 cm³
- eau q.s.p. 4 cm³

## Revendications (Revendications pour l'(les) Etat(s) contractant(s) suivant(s) : suivants : AT, BE, CH, DE, DK, FR, GB, IT, LI, LU, NL, SE)

1. Composés de formule : 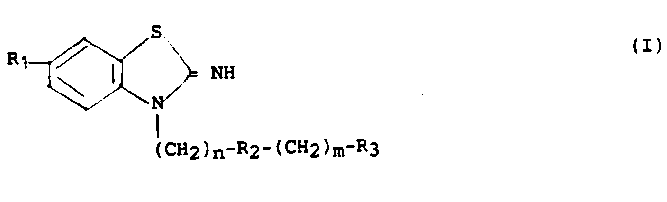 dans laquelle,
- R₁ représente un radical polyfluoroalcoxy,
- R₂ représente un atome de soufre ou d'azote substitué par un radical alkyl ou un radical sulfonyle ou sulfinyle,
- R₃ représente un radical phényle, benzoyle, -NR₄R₅ ou pipéridinyl-4 substitué en position -1 par un radical phénylalkyle,
- R₄ représente un radical alkyle,
- R₅ représente un radical phénylalkyle,
- n est égal à 1,2 ou 3,
- m est égal à 0,1,2 ou 3
étant entendu que les radicaux alkyle et les portions alkyle et alcoxy contiennent 1 à 4 atomes de carbone en chaîne droite ou ramifiée, ainsi que leurs sels avec un acide minéral ou organique.

2. Procédé de préparation des composés de formule (I) selon la revendication 1 pour lesquels R₂ représente un atome de soufre caractérisé en ce que l'on hydrolyse un dérivé de formule: 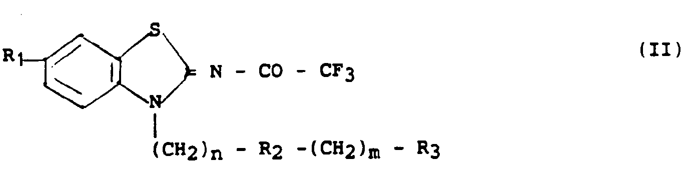 dans laquelle R_{1'} R₃, n et m ont les mêmes significations que dans la revendication 1 et R₂ représente un atome de soufre, isole le produit et le transforme éventuellement en sel avec un acide minéral ou organique.

3. Procédé de préparation des composés de formule (I) selon la revendication 1 pour lesquels R₂ représente un atome de soufre caractérisé en ce que l'on fait réagir un amino-2 polyfluoroalcoxy-6 benzothiazole avec un dérivé de formule : 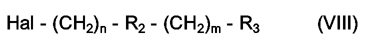 dans laquelle n, met R₃ ont les mêmes significations que dans la formule (I), R₂ représente un atome de soufre, et Hal représente un atome d'halogène, isole le produit et le transforme éventuellement en sel avec un acide minéral ou organique.

4. Procédé de préparation des composés de formule (I) selon la revendication 1 pour lesquels R₂ représente un radical sulfonyle ou sulfinyle caractérisé en ce que l'on oxyde un composé de formule (I) correspondant pour lequel R₂ représente un atome de soufre, isole le produit et le transforme éventuellement en sel avec un acide minéral ou organique.

5. Procédé de préparation des composés de formule (I) selon la revendication 1 pour lesquels R₂ représente un atome d'azote substitué par un radical alkyle caractérisé en ce que l'on fait réagir du brome et un thiocyanate de métal alcalin sur un dérivé de formule : 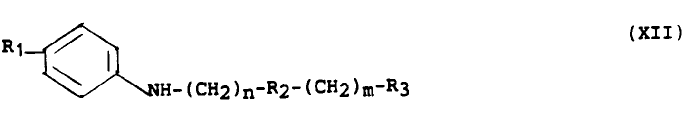 dans laquelle R_{1'} R₃, net m ont les mêmes significations que dans la formule (I) et R₂ représente un atome d'azote substitué par un radical alkyle, isole le produit et le transforme éventuellement en sel avec un acide minéral ou organique.

6. Médicaments caractérisés en ce qu'ils contiennent en tant que principe actif au moins un composé de formule (I) selon la revendication 1.

7. Médicaments selon la revendication 6 pour le traitement des affections où le glutamate est impliqué.

## Revendications (Revendications pour l'(les) Etat(s) contractant(s) suivant(s) : ES, GR)

1. Procédé de préparation des composés de formule : 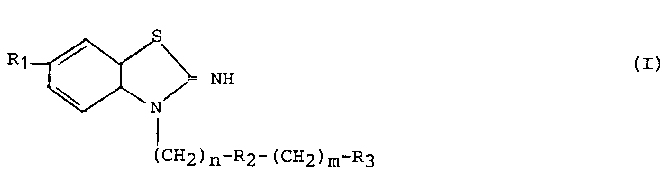 dans laquelle,
- Rᵢ représente un radical polyfluoroalcoxy,
- R₂ représente un atome de soufre ou d'azote substitué par un radical alkyl ou un radical sulfonyle ou sulfinyle,
- R₃ représente un radical phényle, benzoyle, -NR₄R₅ ou pipéridinyl-4 substitué en position -1 par un radical phénylalkyle,
- R₄ représente un radical alkyle,
- R₅ représente un radical phénylalkyle,
- n est égal à 1,2 ou 3,
- m est égal à 0,1,2 ou 3
étant entendu que les radicaux alkyle et les portions alkyle et alcoxy contiennent 1 à 4 atomes de carbone en chaîne droite ou ramifiée, ainsi que leurs sels avec un acide minéral ou organique, caractérisé en ce que :
A. pour la préparation des composés de formule (I) pour lesquels R₂ représente un atome de soufre on hydrolyse un dérivé de formule : 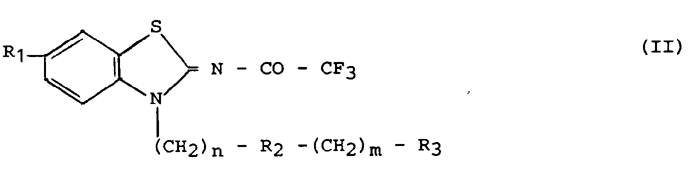 dans laquelle R_{1'} R₃, net m ont les mêmes significations que dans la formule et R₂ représente un atome de soufre, isole le produit et le transforme éventuellement en sel avec un acide minéral ou organique,
B. pour la préparation des composés de formule (I) pour lesquels R₂ représente un atome de soufre on fait réagir un amino-2 polyfluoroalcoxy-6 benzothiazole avec un dérivé de formule : 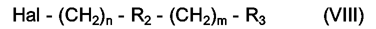 dans laquelle n, met R₃ ont les mêmes significations que dans la formule (I), R₂ représente un atome de soufre, et Hal représente un atome d'halogène, isole le produit et le transforme éventuellement en sel avec un acide minéral ou organique,
C. pour la préparation des composés de formule (I) pour lesquels R₂ représente un radical sulfonyle ou sulfinyle on oxyde un composé de formule (I) correspondant pour lequel R₂ représente un atome de soufre, isole le produit et le transforme éventuellement en sel avec un acide minéral ou organique,
D. pour la préparation des composés de formule (I) pour lesquels R₂ représente un atome d'azote substitué par un radical alkyle on fait réagir du brome et un thiocyanate de métal alcalin sur un dérivé de formule : 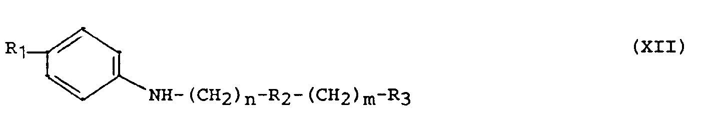 dans laquelle R_{1'} R₃, n et m ont les mêmes significations que dans la formule (I) et R₂ représente un atome d'azote substitué par un radical alkyle, isole le produit et le transforme éventuellement en sel avec un acide minéral ou organique.

## Patentansprüche (Patentansprüche für folgende(n) Vertragsstaat(en) : AT, BE, CH, DE, DK, FR, GB, IT, LI, LU, NL, SE)

1. Verbindungen der Formel 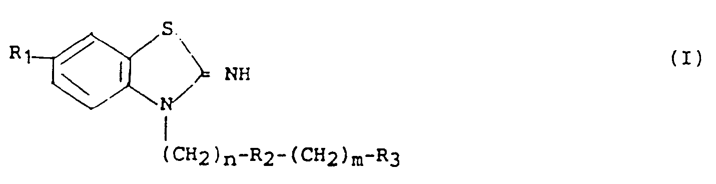 worin
- R₁ für einen Polyfluoralkoxyrest steht,
- R₂ für ein Schwefel-oder Stickstoffatom, das durch einen Alkyl-oder einen Sulfonyl-oder Sulfinylrest substituiert ist, steht,
- R₃ für einen Phenyl-, Benzoyl-, -NR₄R₅-Rest oder einen an Position -1 durch einen Phenylalkylrest substituierten 4-Piperidinylrest steht,
- R₄ für einen Alkylrest steht,
- R₅ für einen Phenylalkylrest steht,
- n den Wert 1, 2 oder 3 hat,
- m den Wert 0, 1, 2 oder 3 hat,
mit der Maßgabe, daß die Alkylreste und die Alkyl- und Alkoxyeinheiten 1 bis 4 Kohlenstoffatome in gerader oder verzweigter Kette enthalten,
sowie ihre Salze mit mineralischen oder organischen Säuren.

2. Verfahren zur Herstellung der Verbindungen der Formel (I) nach Anspruch 1, worin R₂ für ein Schwefelatom steht, dadurch gekennzeichnet, daß man ein Derivat der Formel 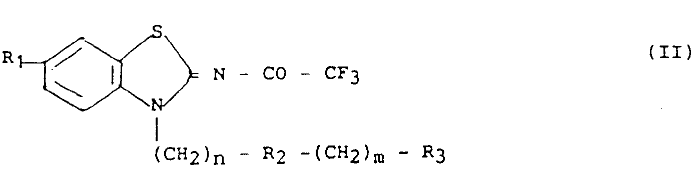 worin R_{1'} R₃, n und m wie unter Anspruch 1 definiert sind, und R₂ für ein Schwefelatom steht, hydrolysiert, die Verbindung isoliert und gegebenenfalls in ein Salz mit einer mineralischen oder organischen Säure überführt.

3. Verfahren zur Herstellung der Verbindungen der Formel (I) nach Anspruch 1, worin R₂ für ein Schwefelatom steht, dadurch gekennzeichnet, daß man 2-Amino-6-polyfluoralkoxybenzothiazol mit einem Derivat der Formel 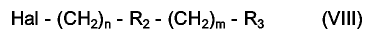 worin n, m und R₃ wie für die Formel (I) definiert sind, R₂ für ein Schwefelatom steht, und Hal für ein Halogenatom steht, umsetzt, die Verbindung isoliert und gegebenenfalls in ein Salz mit einer mineralischen oder organischen Säure überführt.

4. Verfahren zur Herstellung der Verbindungen der Formel (I) nach Anspruch 1, worin R₂ für eine Sulfonyl-oder Sulfinylgruppe steht, dadurch gekennzeichnet, daß man eine entsprechende Verbindung der Formel (I),
worin R₂ für ein Schwefelatom steht, oxidiert, die Verbindung isoliert und gegebenenfalls in ein Salz mit einer mineralischen oder organischen Säure überführt.

5. Verfahren zur Herstellung der Verbindungen der Formel (I) nach Anspruch 1, worin R₂ für ein durch einen Alkylrest substituiertes Stickstoffatom steht, dadurch gekennzeichnet, daß man Brom und ein Alkalimetalthiocyanat mit einem Derivat der Formel 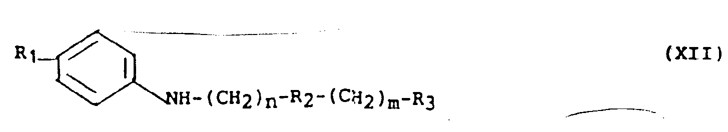 worin R_{1'} R₃, n und m wie für die Formel (I) definiert sind, und R₂ für ein durch einen Alkylrestsubstituiertes Stickstoffatom steht, umsetzt, die Verbindung isoliert und gegebenenfalls in ein Salz mit einer mineralischen oder organischen Säure überführt.

6. Medikamente, dadurch gekennzeichnet, daß sie als Wirkstoff mindestens eine Verbindung der Formel (I) nach Anspruch 1 enthalten.

7. Medikamente nach Anspruch 6 zur Behandlung von Störungen, an denen Glutamat beteiligt ist.

## Patentansprüche (Patentansprüche für folgende(n) Vertragsstaat(en) : ES, GR)

1. Verfahren zur Herstellung von Verbindungen der Formel 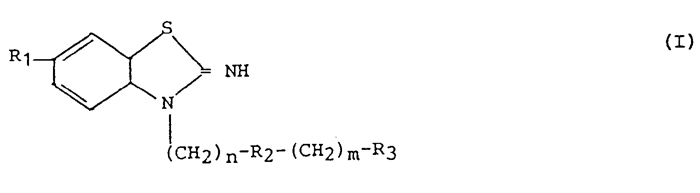 worin
- R₁ für einen Polyfluoralkoxyrest steht,
- R₂ für ein Schwefel- oder Stickstoffatom, das durch einen Alkyl- oder einen Sulfonyl- oder Sulfinylrest substituiert ist, steht,
- R₃ für einen Phenyl-, Benzoyl-, -NR₄R₅-Rest oder einen an Position -1 durch einen Phenylalkylrest substituierten 4-Piperidinylrest steht,
- R₄ für einen Alkylrest steht,
- R₅ für einen Phenylalkylrest steht,
- n den Wert 1, 2 oder 3 hat,
- m den Wert 0, 1, 2 oder 3 hat,
mit der Maßgabe, daß die Alkylreste und die Alkyl- und Alkoxyeinheiten 1 bis 4 Kohlenstoffatome in gerader oder verzweigter Kette enthalten, sowie ihrer Salze mit mineralischen oder organischen Säuren, dadurch gekennzeichnet, daß
A) zur Herstellung der Verbindungen der Formel (I) nach Anspruch 1, worin R₂ für ein Schwefelatom steht, man ein Derivat der Formel 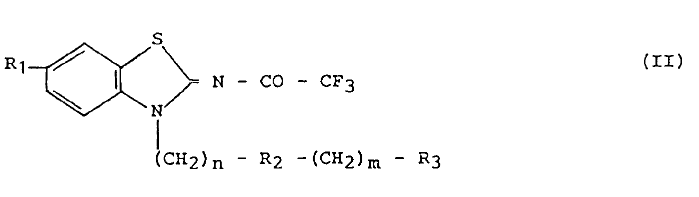 worin R_{1'} R₃, n und m wie für die Formel (I) definiert sind, und R₂ für ein Schwefelatom steht, hydrolysiert, die Verbindung isoliert und gegebenenfalls in ein Salz mit einer mineralischen oder organischen Säure überführt,
B) zur Herstellung der Verbindungen der Formel (I), worin R₂ für ein Schwefelatom steht, man 2-Amino-6-polyfluoralkoxybenzothiazol mit einem Derivat der Formel 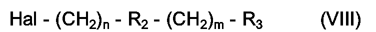 worin n, m und R₃ wie für die Formel (I) definiert sind, R₂ für ein Schwefelatom steht, und Hal für ein Halogenatom steht, umsetzt, die Verbindung isoliert und gegebenenfalls in ein Salz mit einer mineralischen oder organischen Säure überführt,
C) zur Herstellung der Verbindungen der Formel (I), worin R₂ für eine Sulfonyl- oder Sulfinylgruppe steht, man eine entsprechende Verbindung der Formel (I), worin R2 für ein Schwefelatom steht, oxidiert, die Verbindung isoliert und gegebenenfalls in ein Salz mit einer mineralischen oder organischen Säure überführt,
D) zur Herstellung der Verbindungen der Formel (I), worin R2 für ein durch einen Alkylrestsubstituiertes Stickstoffatom steht, man Brom und ein Alkalimetalthiocyanat mit einem Derivat der Formel 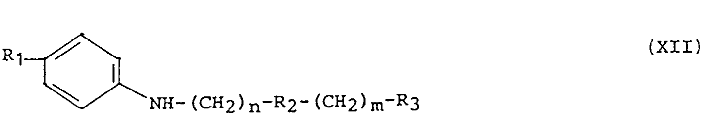 worin R_{1'} R₃, n und m wie für die Formel (I) definiert sind, und R₂ für ein durch einen Alkylrest substituiertes Stickstoffatom steht, umsetzt, die Verbindung isoliert und gegebenenfalls in ein Salz mit einer mineralischen oder organischen Säure überführt.

## Claims (Claims for the following Contracting State(s) : AT, BE, CH, DE, DK, FR, GB, IT, Ll, LU, NL, SE)

1. Compounds of formula: 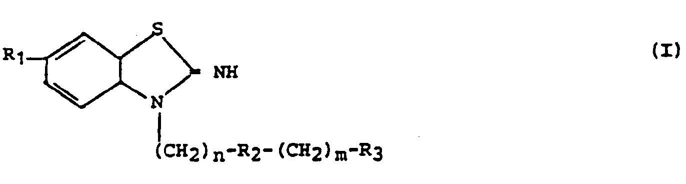 in which
- Rᵢ represents a polyfluoroalkoxy radical,
-R2 represents a sulphur atom or a nitrogen atom substituted with an alkyl radical or a sulphonyl or sulphinyl radical,
- R₃ represents a phenyl, benzoyl or -NR₄R₅ radical or a 4-piperidyl radical substituted in position 1 with a phenylalkyl radical,
- R₄ represents an alkyl radical,
- R₅ represent a phenylalkyl radical,
- n is equal to 1, 2 or 3,
- m is equal to 0, 1, 2 or 3
it being understood that the alkyl radicals and the alkyl and alkoxy portions contain 1 to 4 carbon atoms in a straight or branched chain, as well as their salts with an inorganic or organic acid.

2. Process for the preparation of the compounds of formula (I) according to claim 1 for which R₂ represents a sulphur atom, characterized in that a derivative of formula: 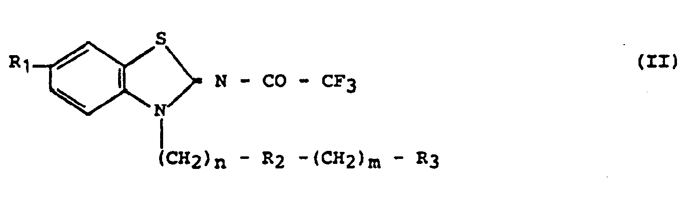 in which R_{1'} R₃, n and m have the same meanings as in claim 1 and R₂ represents a sulphur atom, is hydrolysed, and the product is isolated and optionally converted to a salt with an inorganic or organic acid.

3. Process for the preparation of the compounds of formula (I) according to claim 1 for which R₂ represents a sulphur atom, characterized in that a 2-amino-6-polyfluoroalkoxybenzothiazole is reacted with a derivative of formula: 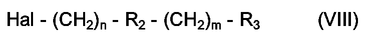 in which n, m and R₃ have the same meanings as in the formula (I), R₂ represents a sulphur atom, and Hal represents a halogen atom, and the product is isolated and optionally converted to a salt with an inorganic or organic acid.

4. Process for the preparation of the compounds of formula (I) according to claim 1 for which R₂ represents a sulphonyl or sulphinyl radical, characterized in that a corresponding compound of formula (I) for which R₂ represents a sulphur atom is oxidized, and the product is isolated and optionally converted to a salt with an inorganic or organic acid.

5. Process for the preparation of the compounds of formula (I) according to claim 1 for which R₂ represents a nitrogen atom substituted with an alkyl radical, characterized in that bromine and an alkali metal thiocyanate are reacted with a derivative of formula: 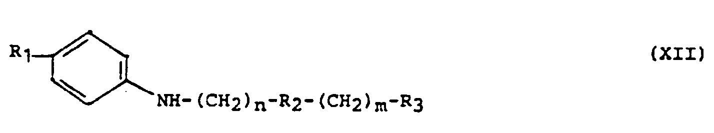 in which R_{1'} R₃, n and m have the same meanings as in the formula (I) and R₂ represents a nitrogen atom substituted with an alkyl radical, and the product is isolated and optionally converted to a salt with an inorganic or organic acid.

6. Medicaments characterized in that they contain as active principle at least one compound of formula (I) according to claim 1.

7. Medicaments according to claim 6 for the treatment of complaints in which glutamate is involved.

## Claims (Claims for the following Contracting State(s) : ES, GR)

1. Process for the preparation of the compounds of formula: 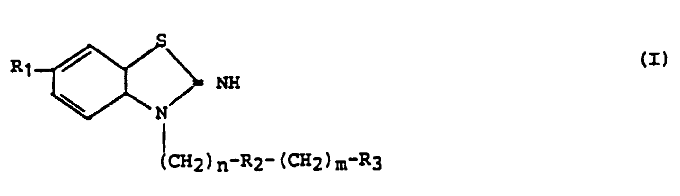 in which
- R₁ represents a polyfluoroalkoxy radical,
- R₂ represents a sulphur atom or a nitrogen atom substituted with an alkyl radical or a sulphonyl or sulphinyl radical,
- R₃ represents a phenyl, benzoyl or -NR₄R₅ radical or a 4-piperidyl radical substituted in position 1 with a phenylalkyl radical,
- R₄ represents an alkyl radical,
- R₅ represent a phenylalkyl radical,
- n is equal to 1, 2 or 3,
- m is equal to 0, 1, 2 or 3 it being understood that the alkyl radicals and the alkyl and alkoxy portions contain 1 to 4 carbon atoms in a straight or branched chain, as well as their salts with an inorganic or organic acid, characterized in that:
A. for the preparation of the compounds of formula (I) for which R₂ represents a sulphur atom, a derivative of formula: 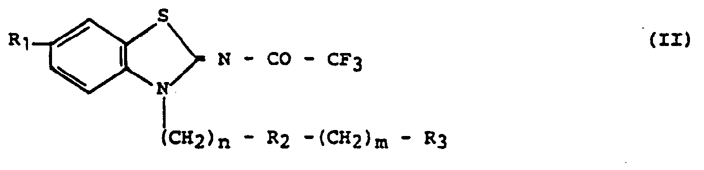 in which R_{1'} R₃, n and m have the same meanings as in the formula (I) and R₂ represents a sulphur atom, is hydrolysed, and the product is isolated and optionally converted to a salt with an inorganic or organic acid,
B. for the preparation of the compounds of formula (I) for which R₂ represents a sulphur atom, a 2-amino-6-polyfluoroalkoxybenzothiazole is reacted with a derivative of formula: 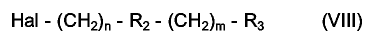 in which n, m and R₃ have the same meanings as in the formula (I), R₂ represents a sulphur atom, and Hal represents a halogen atom, and the product is isolated and optionally converted to a salt with an inorganic or organic acid,
C. for the preparation of the compounds of formula (I) for which R₂ represents a sulphonyl or sulphinyl radical, a corresponding compound of formula (I) for which R₂ represents a sulphur atom is oxidized, and the product is isolated and optionally converted to a salt with an inorganic or organic acid,
D. for the preparation of the compounds of formula (I) for which R₂ represents a nitrogen atom substituted with an alkyl radical, bromine and an alkali metal thiocyanate are reacted with a derivative of formula: 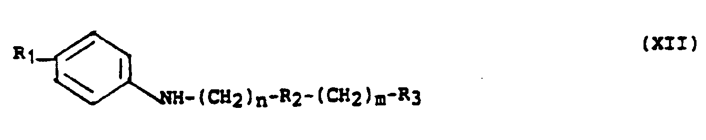 in which R_{1'} R₃, n and m have the same meanings as in the formula (I) and R₂ represents a nitrogen atom substituted with an alkyl radical, and the product is isolated and optionally converted to a salt with an inorganic or organic acid.
